(19) Europäisches Patentamt
European Patent Office
Office européen des brevets

(11) Publication number: 0 058 009
A1

(12) EUROPEAN PATENT APPLICATION

(21) Application number: 82300377.7

(22) Date of filing: 26.01.82

(51) Int. Cl.³: C 07 C 103/76
A 61 K 31/165

(30) Priority: 28.01.81 JP 10135/81
28.01.81 JP 10136/81

(43) Date of publication of application:
18.08.82 Bulletin 82/33

(84) Designated Contracting States:
DE FR GB

(71) Applicant: KYOWA HAKKO KOGYO CO., LTD.
Ohtemachi Bldg., 6-1 Ohtemachi Itchome
Chiyoda-ku Tokyo 100(JP)

(72) Inventor: Hirayama, Tatsuyuki
410-1, Nameri Nagaizumi-cho
Sunto-gun Shizuoka-ken(JP)

(72) Inventor: Shuto, Katsuichi
410-1, Nameri Nagaizumi-cho
Sunto-gun Shizuoka-ken(JP)

(72) Inventor: Naruse, Masao
870-51, Kamitsuchidana Ayase-shi
Kanagawa-ken(JP)

(72) Inventor: Kayashi, Chihiro
494, Omiya-cho Matsuta Tsurumi-ku
Osaka-shi Osaka-fu(JP)

(72) Inventor: Murata, Akira
701-Naka 2-242 Uchiage Neyagawa-shi
Osaka-fu(JP)

(74) Representative: Lambert, Hugh Richmond et al,
D. YOUNG & CO. 10 Staple Inn
London, WC1V 7RD(GB)

(54) Novel benzanilide derivatives and pharmaceutical compositions containing them.

(57) Derivatives of benzanilide are disclosed having analgesic properties as well as pharmaceutical preparations containing them:

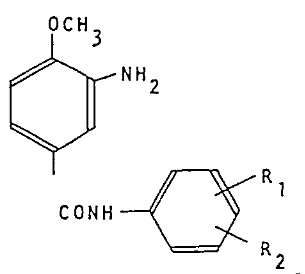

wherein $R_1$ and $R_2$ represent H, $C_1$-$C_5$ alkyl or $CF_3$; and acid addition salts thereof.

EP 0 058 009 A1

# NOVEL BENZANILIDE DERIVATIVES AND PHARMACEUTICAL COMPOSITIONS CONTAINING THEM

This invention relates to derivatives of benzanilide having analgesic properties and to pharmaceutical compositions containing them. More particularly the invention is based on the discovery that compounds of formula I *infra*, and their acid addition salts have anti-inflammatory and analgesic properties and, moreover, are of extremely low toxicity:

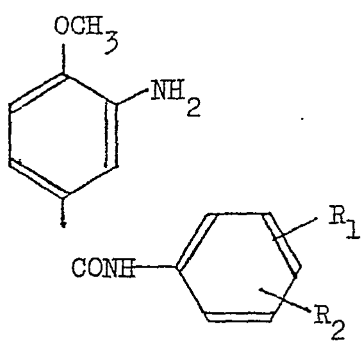

(I)

wherein $R_1$ and $R_2$ are the same or different groups selected from hydrogen, halogen, $C_1$-$C_5$ alkyl and trifluoromethyl.

Some of the compounds represented by the general formula (I) are known compounds which have been disclosed in Japanese Published Examined Patent Application Nos. 7527/56 and 3941/63. However, in these applications, there is a description that benzanilide derivatives may be used as starting materials for dyestuffs, whereas there is neither description nor suggestion that these compounds are used as analgesic agents.

Accordingly, in one aspect the present invention provides a pharmaceutical composition comprising a pharmaceutically acceptable carrier and, as pharmaceutically active ingredient, a

compound of formula I, or a pharmaceutically acceptable acid addition salt thereof. In a second aspect, the invention provides a novel group of benzanilide derivatives, being compounds of formula Ia:

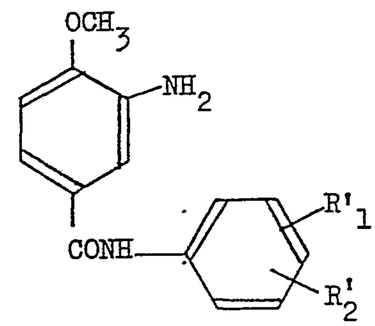

(Ia)

wherein $R'_1$ represents a fluorine atom or a trifluoromethyl group and $R'_2$ represents a hydrogen atom, a halogen atom or a trifluoromethyl group; and the pharmaceutically acceptable acid addition salts thereof.

In the definition of $R_1$ or $R_2$, and $R'_1$ or $R'_2$ in the formulae (1) and (1a), the term "halogen atom" represents a chlorine, a fluorine, a bromine or an iodine atom; and the term "alkyl group having 1 to 5 carbon atoms" represents a methyl, an ethyl, a propyl, a butyl, or a pentyl group.

Suitable acid addition salts in accordance with this invention include inorganic acid addition salts such as hydrochloride, sulfate, phosphate, hydrobromide, etc. or organic acid addition salts such as an acetate, propionate, maleate, fumarate, tartrate, citrate, etc.

The compounds of formula (I) may be produced, for example, the following process (Steps 1 and 2):

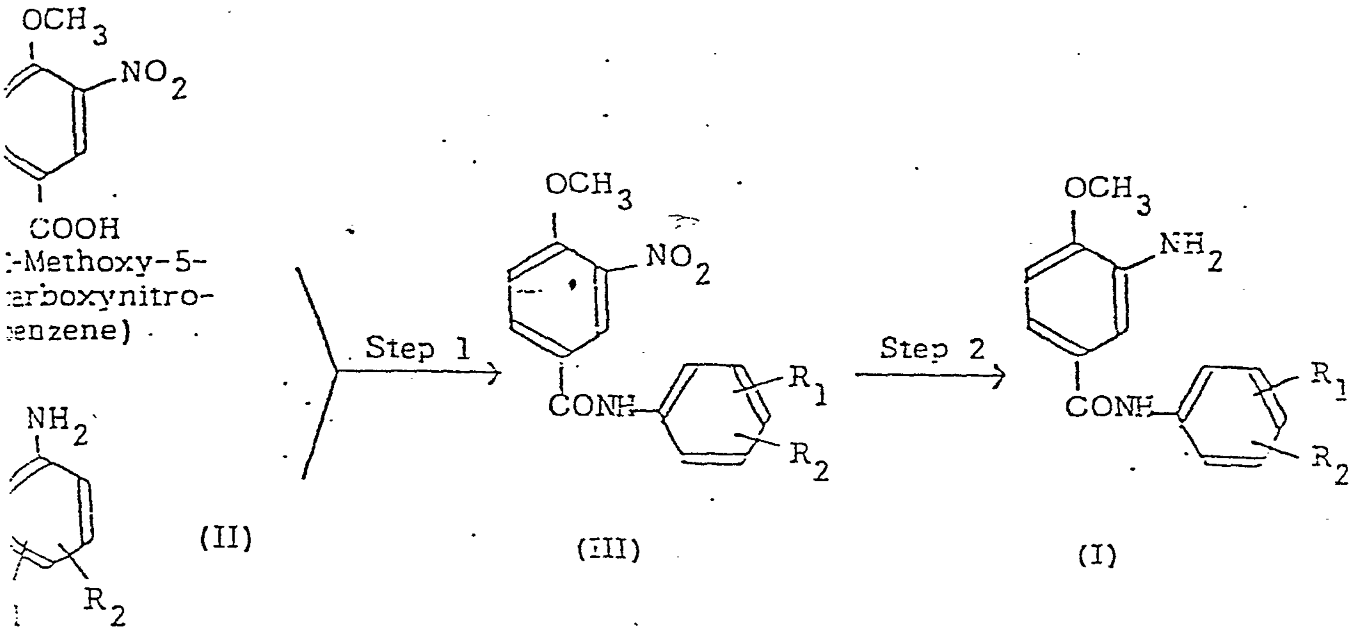

:-Methoxy-5-
:arboxynitro-
:enzene)

:iline
:rivative)

(II) (III) (I)

herein $R_1$ and $R_2$ have the same meaning as defined above. Each step : described in more detail.

:ep 1

The compound of the general formula (III) is obtained by :ndensing 2-methoxy-5-carboxynitrobenzene and the aniline derivative : the general formula (II) in a solvent.

As the aniline derivative, there may be employed fluoroaniline, :ifluoromethylaniline, ditrifluoromethylaniline, chloro-trifluoromethylaniline, :c.

The aniline derivative is used in an equimolar or a greater amount ased on 2-methoxy-5-carboxynitrobenzene; generally 1 - 3 moles of the :iline derivative per mole of 2-methoxy-5-carboxynitrobenzene.

BAD ORIGINAL

As the condensing agent used during this step, there may be employed phosphorus oxychloride, phosphorus pentoxide, phosphorus trioxide, thionyl chloride, sulfuryl chloride, etc.

As the solvent, there may be employed toluene, xylene, tetrachloroethylene, trichloroethylene, nitrobenzene, etc.

The condensation reation is generally completed in 1 - 24 hours under reflux at temperatures from 30 to 250 °C.

After completion of the reaction, isolation of the desired compound from the reaction mixture is conducted according to conventional practice of synthetic chemistry. Generally, after cooling the reaction mixture to room temperature, the solvent is separated by steam distillation, and filtration is conducted to obtain a crude desired compound. Further, said crude desired compound is purified by recrystallization, column chromatography, preparative thin layer chromatography, etc. to obtain a pure desired compound.

Step 2

The desired compound of the general formula (I) is obtained by reducing the compound III.

As the reducing agent, there may be employed sodium disulfide, metal (iron, zinc, etc.) and acid (hydrochloric acid, acetic acid, etc.).

As the method for reduction, catalytic reduction using hydrogen (catalyst: Raney nickel, palladium-carbon, etc.) may be conducted. As the solvent, there may be employed water, alcohols (methyl alcohol, ethyl alcohol, isopropyl alcohol, etc.) etc.

The reduction reaction is generally completed in 1 - 10 hours at 50 - 150°C.

After completion of the reaction, isolation of the desired compound from the reaction mixture is conducted according to conventional practice of synthetic chemistry. Generally, after removing the reducing agent, etc., from the reaction mixture, concentration, neutralization, or the like, is conducted to obtain a crude desired compound. Further, said crude desired compound is purified by recrystallization, column chromatography, preparative thin layer chromatography, etc., to obtain a pure desired compound.

The thus obtained compound may be further derived, according to a conventional manner, to an inorganic acid addition salt, such as a hydrochloride, sulfate, phosphate, hydrobromide, etc., or an organic acid addition salt such as an acetate, propionate, maleate, fumarate, tartrate, citrate, etc.

Representative compounds useful in the present invention and physicochemical properties thereof are given in Table 1.

Table 1

| Compound No. | Name | Structural Formula | m.p. (°C) | Elemental Analysis Calc'd (%) | Found (%) |
|---|---|---|---|---|---|
| 1 | 3-Amino-3'-fluoro-4-methoxybenzanilide | 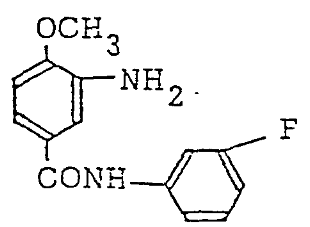 | 140.3 - 140.8 | C: 64.61<br>H: 5.03<br>N: 10.76 | 64.74<br>5.02<br>10.69 |
| 2 | 3-Amino-3'-trifluoromethyl-4-benzanilide | 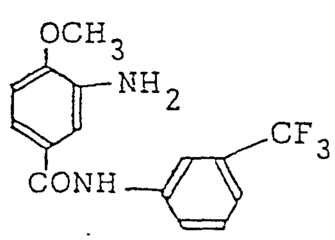 | 150.7 - 151.8 | C: 58.07<br>H: 4.22<br>N: 9.03 | 57.99<br>4.26<br>8.95 |

Table 1 (Continued)

| Compound No. | Name | Structural Formula | m.p. (°C) | Elemental Calc'd (%) | Analysis Found (%) |
|---|---|---|---|---|---|
| 3 | 3-Amino-3',5'-ditrifluoromethyl-4-methoxybenzanilide | 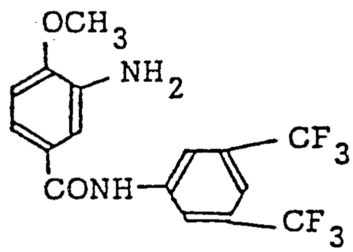 | 199.7 - 200.4 | C: 50.80<br>H: 3.20<br>N: 7.40 | 50.74<br>3.13<br>7.43 |
| 4 | 3-Amino-2'-trifluoromethyl-4'-chloro-4-methoxybenzanilide | 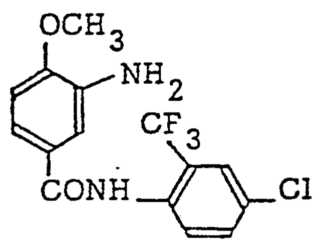 | 153.5 - 154.0 | C: 52.26<br>H: 3.51<br>N: 8.13 | 52.26<br>3.38<br>8.24 |
| 5 | 3-Amino-2'-chloro-5'-trifluoromethyl-4-methoxybenzanilide | 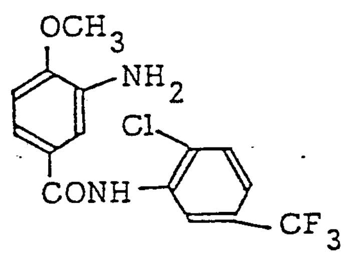 | 163.1 - 163.6 | C: 52.26<br>H: 3.51<br>N: 8.13 | 52.25<br>3.36<br>7.90 |
| 6 | 3-Amino-4-methoxybenzanilide | 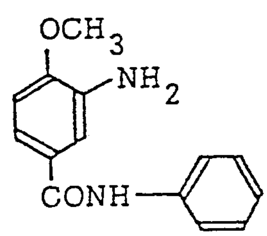 | 153.5 - 154.5 | C: 69.41<br>H: 5.81<br>N: 11.56 | -<br>-<br>- |
| 7 | 3-Amino-2',4'-dichloro-4-methoxybenzanilide | 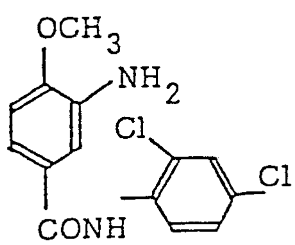 | 155.1 - 155.6 | C: 54.04<br>H: 3.89<br>N: 9.00 | 54.28<br>3.74<br>9.22 |

Table 1 (Continued)

| Compound No. | Name | Structural Formula | m.p. (°C) | Elemental Calc'd (%) | Analysis Found (%) |
|---|---|---|---|---|---|
| 8 | 3-Amino-2',6'-dimethyl-4-methoxy-benzanilide | 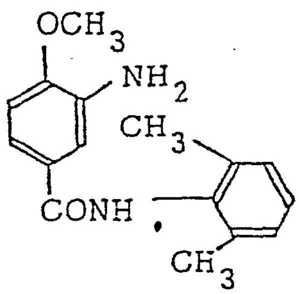 | 198.6 - 199.5 | C: 71.09<br>H: 6.71<br>N: 10.36 | 69.47<br>6.56<br>10.11 |

NOTE: The compounds of Compound Nos. 1-5 are novel compounds. The compounds of Compound Nos. 6-8 are known compounds which are disclosed in Japanese Published Examined Patent Application Nos. 7527/56 and 3941/63.

In the description hereinafter, the compounds of Compound Nos. 1, 2, 3, 4, 5, 6, 7 and 8 are referred to as Compounds 1, 2, 3, 4, 5, 6, 7 and 8, respectively.

Figures 1, 2, 3, 4 and 5 show infrared absorption spectra of Compounds 1, 2, 3, 4 and 5, respectively.

Next, the acute toxicity, anti-inflammatory effect and analgesic effect of some specific compounds among the compounds of the general formula (I) are explained. As the control compounds, Mepirizole [4-methoxy-2-(5-methoxy-3-methylpyrazol-1-yl)-6-methylpyrimidine] and Ibuprofen [α-methyl-4-(2-methylpropyl) benzene acetic acid] are used in each test.



BAD ORIGINAL

(1) Acute Toxicity

Groups of 2 - 6 dd strain male mice weighing 20 ± 1 g were used, the compound to be tested was administered orally, and the mice were observed for a week to count the number of the deaths. The results are given in Table 2.

(2) Effect on carrageenin-induced paw edema (Anti-inflammatory Effect)

This test was conducted according to the method by Winter et al.[Winter C.A. et al.: Proc. Soc. Exptl. Biol. Med. 111, 544 (1962)]. As the animals to be tested, groups of 5 - 10 Wistar strain male rats weighing 140 ± 10 g were used, and a 1% carrageenin solution was employed as the phlogistic agent. The compound to be tested was orally administered to the animals, and an hour later, 0.1 ml of the phlogistic agent was injected subcutaneously to the right hind paw, to induce edema.

The volume of the paw was measured before the injection of the phlogistic agent and 3 hours after the injection, and based on these volume measurements, the edema rate was calculated from the following equation:

$$\text{Edema Rate } (\%) = \frac{Vt - Vn}{Vn} \times 100$$

Vn: The volume before the phlogistic agent treatment.

Vt: The volume after the phlogistic agent treatment.

The effect was determined, compared with the control group, the group to which the compound to be tested was not administered and expressed in terms of the inhibition rate of edema. The inhibition rate was calculated from the following equation. The results are also given in Table 2.

$$\text{Inhibition Rate (\%)} = \frac{Ec - Et}{Ec} \times 100$$

Ec: The average edema rate of the control group.

Et: The average edema rate of the group to which the compound to be tested was administered.

(3) Analgesic Effect (Acetic Acid Writhing Method)

This was done according to the method by Koster, et al. [Federation Proc. 18, 412 (1959)].

The animals to be tested were groups of 10 dd strain male mice weighing 20 ± 1 g.

The compound to be tested was administered orally, and 0 minutes after the administration, 0.2 ml of a 0.7% acetic acid solution was administered intraperitoneally. For a period of 10 minutes from 0 minutes after the administration of said acetic acid solution, the number of writhes was measured and the average number was determined. The inhibition rate (%) was calculated from the following equation. The results are also given in Table 2.

$$\text{Inhibition Rate (\%)} = \frac{Ec - Et}{Ec} \times 100$$

Ec: The average number of writhes in the control group.

Et: The average number of writhes in the group to which the compound to be tested was administered.



BAD ORIGINAL

Table 2

| Compound to be Tested | | Acute Toxicity Nos. of Deaths/ Nos. of Tests (Dose: 1500mg/kg) | Anti-inflammatory Effect Inhibition Rate (%) (Dose: 100 mg/kg) | Analgesic Effect Inhibition Rate (%) (Dose: 100 mg/kg) |
|---|---|---|---|---|
| Compound | 1 | 0/6 | 64.3 | 41.9 |
| | 2 | 0/6 | 38.8 | 48.8 |
| | 3 | 0/6 | 16.9 | 24.6 |
| | 4 | 0/6 | 8.7 | 34.8 |
| | 5 | 0/6 | 0 | 52.3 |
| | 6 | 0/5 | 55.9 | 43.4 |
| | 7 | 0/6 | 5.5 | 40.2 |
| | 8 | 0/6 | 19.5 | 45.7 |
| Mepirizole | | 2/2 | 29.2 | 44.6 |
| Ibuprofen | | 2/5 | 68.4 | 16.3 |

As is apparent from Table 2, the compounds of the general formula (I) are compounds having low acute toxicity, and excellent anti-inflammatory and/or analgesic effects.

The compounds of the general formula (I) may be employed in the form of tablets, powders, capsules or the like by conventional formulation.

On this occasion, conventional excipients, disintegrants, binders, lubricants, etc., are employed.

Preferably employed excipients include lactose, starch, mannite, etc., disintegrants include calcium carboxymethylcellulose, sodium alginate, etc., lubricants include calcium stearate, magnesium stearate, talc, etc., and binders include polyvinylpyrrolidone, gelatin, etc. The

method for administration is conducted by the oral route, the effective dosage of the compound of the general formula (I) is 50 - 200 mg/ adult human per dose and the number of doses is 3 or 4 times a day.

Certain specific embodiments of the invention are illustrated by the following representative examples.

Example 1

Step 1

17 g of 2-methoxy-5-carboxynitrobenzene, 10 g of 3-fluoroaniline and 68 g of toluene are put into a 300 ml four-necked flask, and the temperature is raised with stirring. After raising the temperature of the mixed liquid to 100 - 110°C, 6.6 g of phosphorus oxychloride is added dropwise over 30 minutes. After the addition, the reaction is conducted under reflux at 110 - 115°C for 6 hours. After completion of the reaction, soda ash is added to the reaction mixture until it becomes alkaline (pH 10 or higher). Thereafter, the toluene and unreacted 3-fluoroaniline are recovered by steam distillation. After completion of the steam distillation, the reaction mixture is cooled to 20°C, and the precipitated crude compound is filtered out. The crude compound is washed with water until the washing becomes neutral, and then said crude compound is dried. Thereafter, it is recrystallized from dioxane to obtain 18.7 g (yield 74.8%) of 3-nitro-3'-fluoro-4-methoxy-benzanilide.

Step 2

18.7 g of the 3-nitro-3'-fluoro-4-methoxy-benzanilide obtained in Step 1, 16.2 g of sodium disulfide and 150 g of water are put into a 300 ml four-necked flask, and the reaction is conducted under reflux for 3 hours. After completion of the reaction, the reaction mixture is cooled to 20°C, and the precipitated crude compound is

filtered out. The crude compound is washed with water until the washing becomes neutral, and then the crude compound is dried. Thereafter, the compound is recrystallized from dioxane to obtain 11.9 g of 3-amino-3'-fluoro-4-methoxy-benzanilde.

The physicochemical properties of this compound are as follows:

Elemental Analysis: For $C_{14}H_{13}O_2N_2F$

| | | | |
|---|---|---|---|
| Calc'd (%): | C: 64.61 | H: 5.03 | N: 10.76 |
| Found (%): | 64.74 | 5.02 | 10.69 |

Melting point (°C): 140.3 - 140.8°C

Examples 2 - 5

Similar procedures to in Example 1 are followed, except that the aniline derivatives indicated in Table 3 are employed instead of 3-fluoroaniline in Example 1, to obtain the results given in Table 3. The physicochemical properties of the obtained compounds are shown in Table 1.

Table 3

| Example | Aniline Derivative | | Desired Compound | |
|---|---|---|---|---|
| | Compound | Amount used (g) | Amount obtained (g) | Yield (%) |
| 2 | 3-Trifluoromethylaniline | 13.8 | 14.9 | 56 |
| 3 | 3,5-Ditrifluoromethylaniline | 19.7 | 19.2 | 59 |
| 4 | 2-Trifluoromethyl-4-chloroaniline | 16.8 | 18.1 | 61 |
| 5 | 2-Chloro-5-trifluoromethylaniline | 16.8 | 16.9 | 57 |

Example 6: Tablets

Ingredients (10,000 tablets)

| | |
|---|---|
| Compound 1 | 500 (g) |
| Lactose | 250 |
| Calcium carboxymethylcellulose | 25 |
| Polyvinylpyrrolidone | 20 |
| Calcium stearate | 10 |
| | 805 (g) |

The Compound 1, lactose and calcium carboxymethylcellulose are weighed in the amounts described above, and made into a uniform mixed powder in a mixer. Thereafter, the uniform mixed powder is made into granules by the wet granulation method using the polyvinylpyrrolidone as the binder. The granules are mixed with the magnesium stearate and compressed to produce tablets.

The weight per tablet is 100 mg.

Example 7: Capsules

Ingredients (10,000 capsules)

| | |
|---|---|
| Compound 2 | 500 (g) |
| Potato starch | 200 |
| Magnesium stearate | 36 |
| Talc | 36 |
| | 772 (g) |

The above-described amounts of the respective ingredients are taken into a mixer and mixed uniformly. This mixed powder is filled into capsules.

The content per capsule is 77.2 mg.

Example 8: Powder

| | |
|---|---|
| Compound 6 | 50 (g) |
| Mannite | 950 |
| | 1,000 (g) |

The above-described amounts of the respective ingredients are taken into a mixer and mixed uniformly.

CLAIMS

1. A pharmaceutical composition comprising a pharmaceutically acceptable carrier and, as an pharmaceutically active ingredient, a compound of the formula:

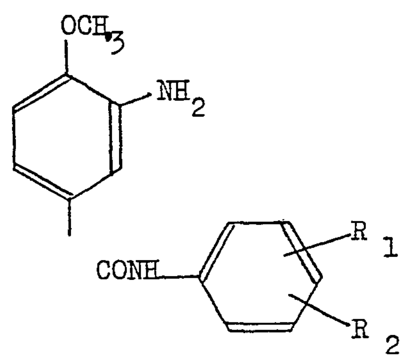

wherein $R_1$ and $R_2$ are the same or different groups selected from hydrogen, halogen, $C_1$-$C_5$ alkyl and trifluoromethyl; or a pharmaceutically acceptable acid addition salt thereof.

2. A composition according to claim 1, in capsule, powder or tablet form.

3. Compounds of the formula:

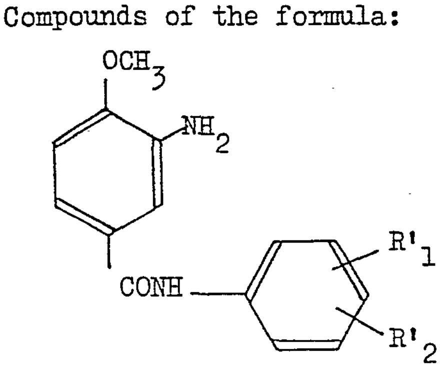

wherein $R'_1$ represents fluorine or trifluoromethyl and $R'_2$ represents hydrogen, halogen or trifluoromethyl, and their pharmaceutically acceptable acid addition salts.

Fig. 1

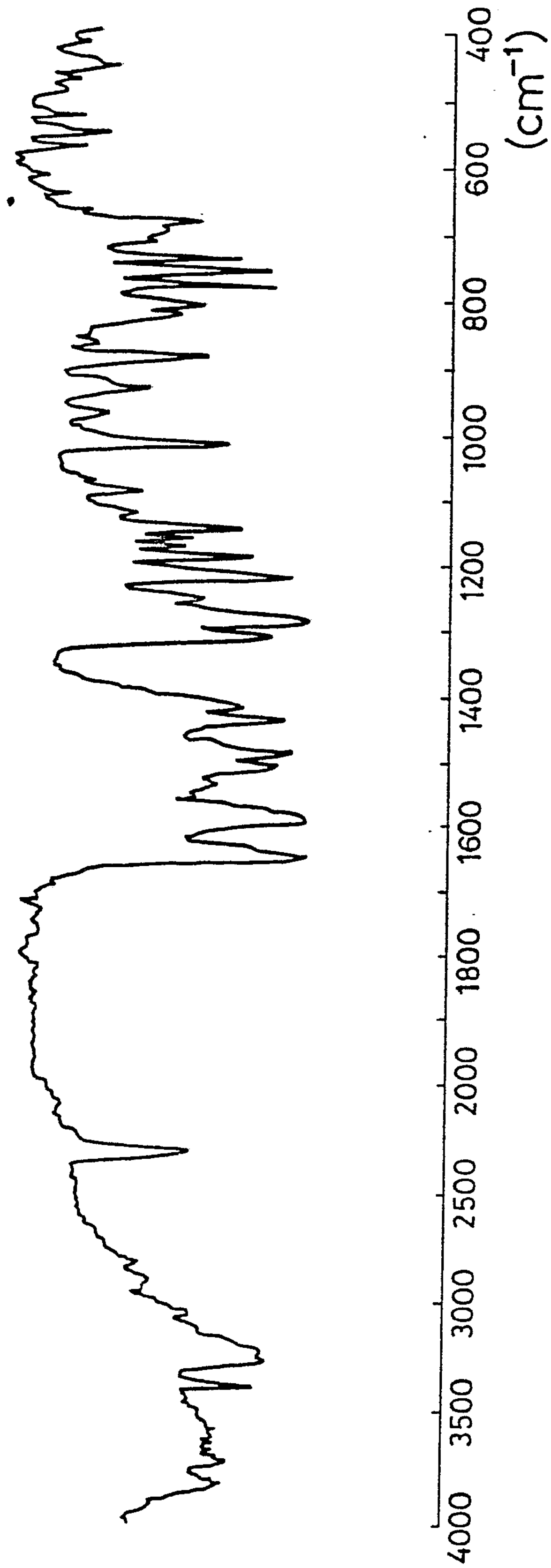

4000
3500
3000
2500
2000
1800
1600
1400
1200
1000
800
600
400
(cm⁻¹)

0058009

2/5

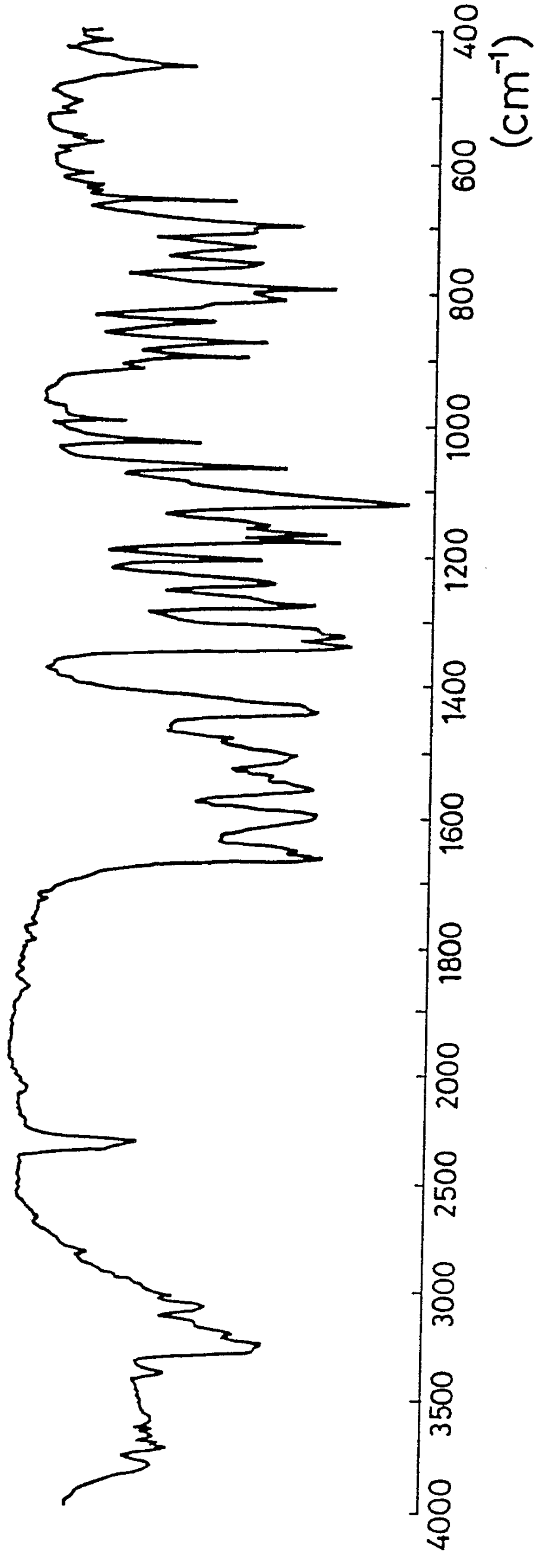


Fig. 2

Fig. 3

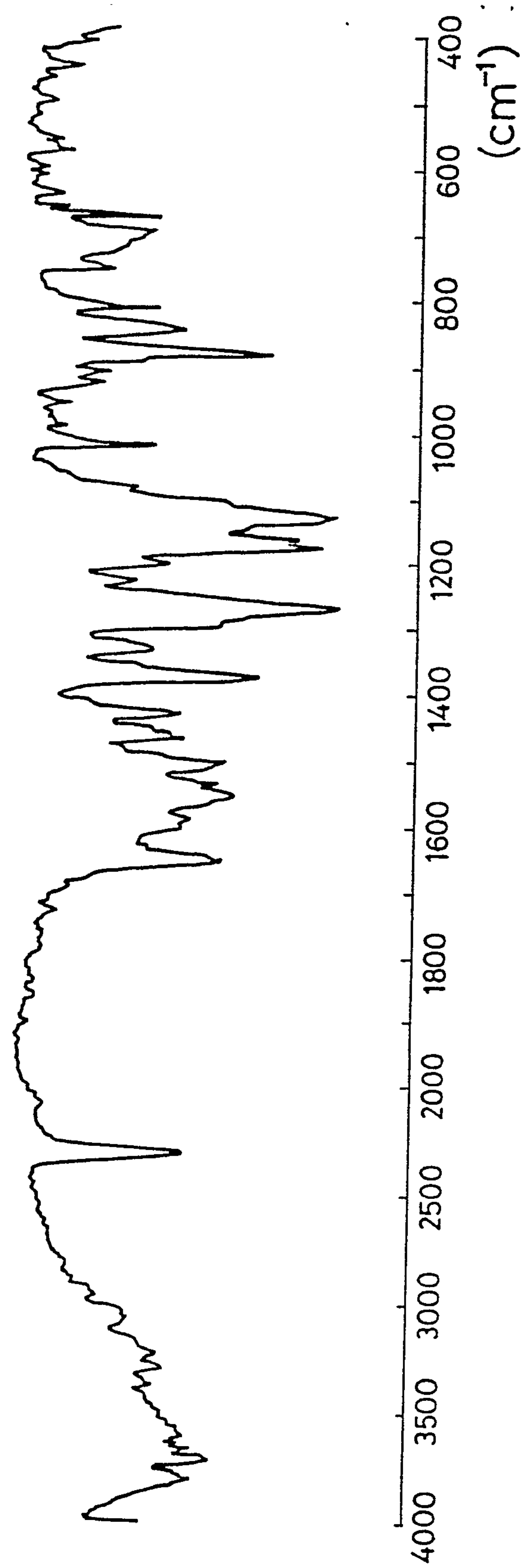

400
600
800
1000
1200
1400
1600
1800
2000
2500
3000
3500
4000
(cm⁻¹)

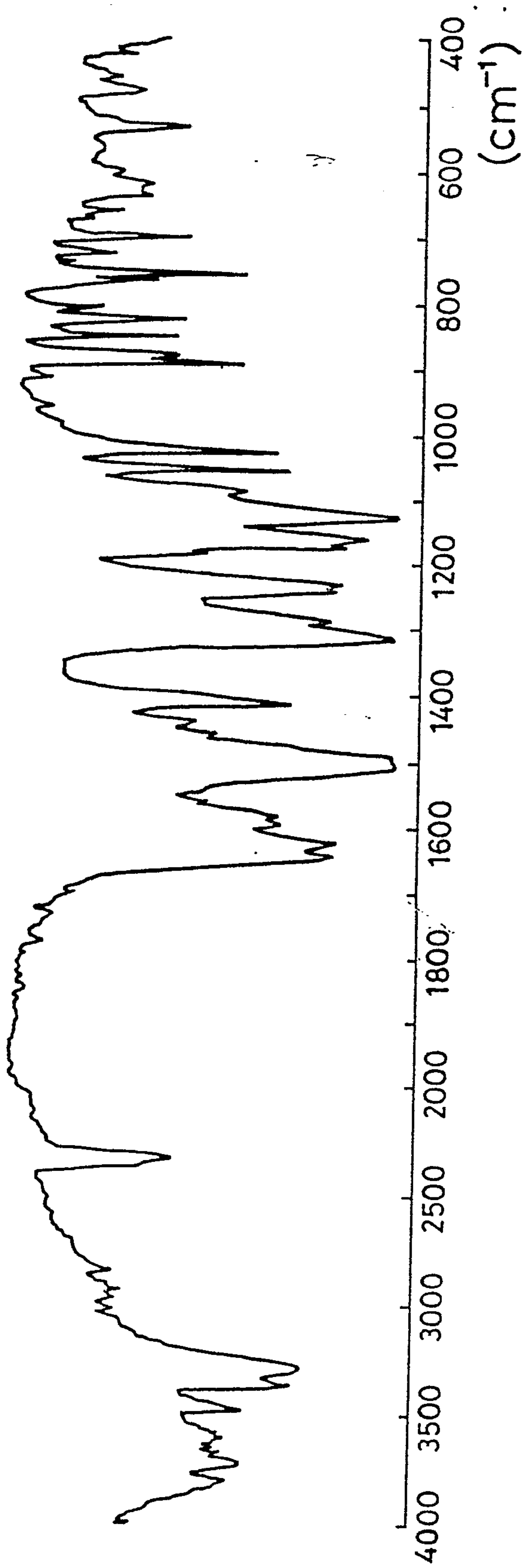
400
600
800
1000
1200
1400
1600
1800
2000
2500
3000
3500
4000
(cm⁻¹)

Fig. 4

Fig. 5

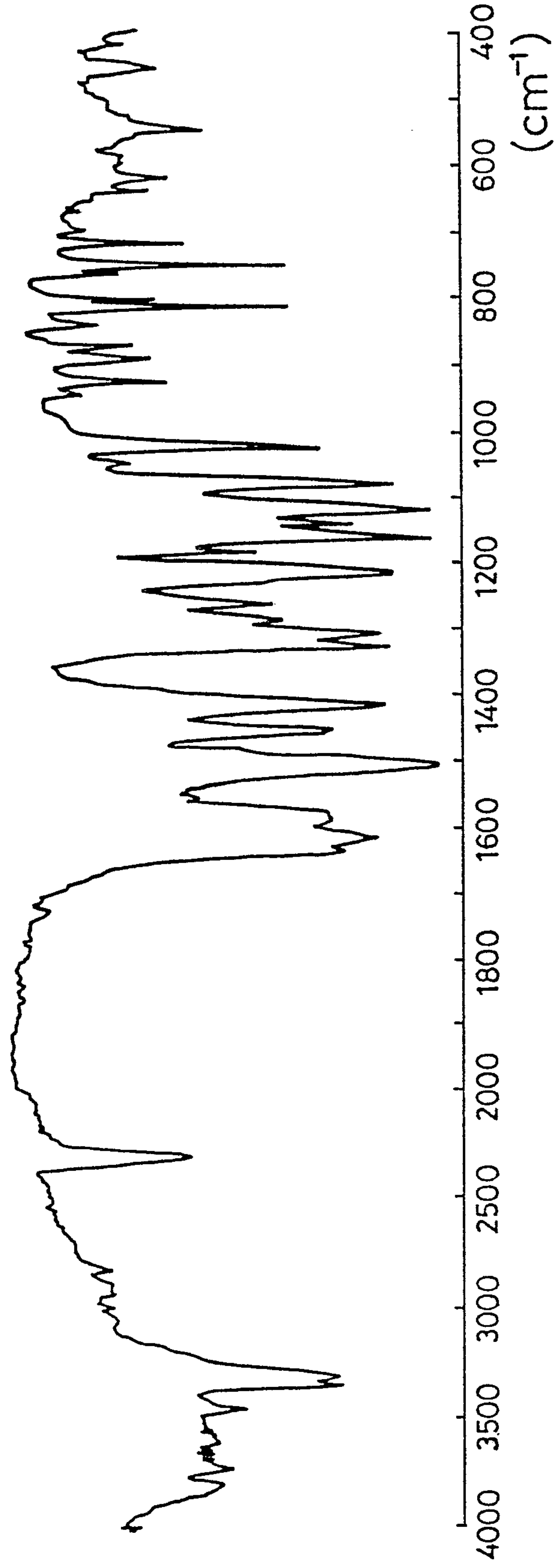
4000
3500
3000
2500
2000
1800
1600
1400
1200
1000
800
600
400
(cm$^{-1}$)

European Patent Office

**EUROPEAN SEARCH REPORT**

0058009

Application number

EP 82 30 0377.7

## DOCUMENTS CONSIDERED TO BE RELEVANT

| Category | Citation of document with indication, where appropriate, of relevant passages | Relevant to claim |
|---|---|---|
| X | GB - A - 1 467 507 (HOECHST AG)<br>* page 5, compound 16 *<br>-- | 3 |
| A | US - A - 4 032 635 (H. UMEZAWA et al.)<br>-- | |
| A | Patent Abstracts of Japan<br>Vol. 1, No. 28, 28 March 1977<br>page 1486C76<br>& JP - A - 51 - 146432<br>---- | |

CLASSIFICATION OF THE APPLICATION (Int. Cl. 3)

C 07 C 103/76
A 61 K 31/165

TECHNICAL FIELDS SEARCHED (Int.Cl. 3)

A 61 K 31/165
C 07 C 103/76

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| Berlin | 01-04-1982 | PHILLIPS |

EPO Form 1503.1 06.78